# EUROPÄISCHE PATENTSCHRIFT

(11) **EP 0 692 962 B1**
(45) Veröffentlichungstag und Bekanntmachung des Hinweises auf die Patenterteilung: **04.03.1998**
(21) Anmeldenummer: 94912437.4
(22) Anmeldetag: 30.03.1994
(51) Int. Cl.: A61K 31/00, A61K 31/135

(54) **VERWENDUNG VON STEROID-ANTAGONISTEN ZUR THERAPIE UND PROPHYLAXE VON DEMENZ-ERKRANKUNGEN**
USE OF STEROID ANTAGONISTS FOR THE THERAPY AND PROPHYLAXIS OF DEMENTIAL ILLNESSES
UTILISATION D'ANTAGONISTES STERO DIENS POUR LE TRAITEMENT ET LA PROPHYLAXIE DE MALADIES DU TYPE DEMENCE

(30) Priorität: 10.04.1993 DE 4311870
(43) Veröffentlichungstag der Anmeldung: 24.01.1996
(60) Teilanmeldung: 97100386.8
(73) Patentinhaber: Altramed Holdings Ltd., Hamilton HM11 (BM)
(72) Erfinder: Denecke, Rainer, 20149 Hamburg (DE)
(74) Vertreter: Klickow, Hans-Henning
(86) Internationale Anmeldenummer: DE9400366
(87) Internationale Veröffentlichungsnummer: WO9423708

(56) Entgegenhaltungen:
- I R CS MED SCI-CLIN. MED. Bd. 4, Nr. 3 , 1976 Seite 113 G. DELITALA ET AL 'Pituitary-gonadal response to clomiphene citrate in Parkinsonism.'
- INT. J. GERIATR. PSYCHIATRY Bd. 2, Nr. 2 , 1987 Seiten 125 - 126 J.J. OHRAM ET AL. 'Treatment of senile dementia with thyrotrophin releasing hormone and clomiphene.'
- J. NATL. CANCER INST. Bd. 85, Nr. 14 , 1993 Seiten 1110 - 1111 S. JENKS '"Hurry up and wait" characterizes RU486 today.'
- PROG. NEURO-PSYCHOPHARMACOL. Bd. 4, Nr. 6 , 1980 Seiten 561 - 567 J. LIBIGER ET AL. 'Drug induced dementia.'

## Beschreibung

Die Erfindung betrifft die Verwendung eines Antiöstrogens zur Herstellung eines Arzneimittels zur Therapie und Prophylaxe von Demenz-Erkrankungen, insbesondere zur Therapie und Prophylaxe von regressiven Zellveränderungen.

Das Auftreten derartiger Erkrankungen wird mit steigendem Lebensalter wahrscheinlicher. Dies hat zur Folge, daß mit steigender Lebenserwartung und einem hieraus resultierenden wachsenden Anteil älterer Menschen an der Bevölkerung die Prävalenz altersbedingter Demenz-Erkrankungen permanent zunimmt. Alterungserscheinungen im Bereich des zentralen Nervensystems werden von einer Vielzahl struktureller und funktioneller Alterationen begleitet. Dies sind beispielsweise Hirnschrumpfung und Gehirngewichtsverlust, Nervenzelldegenerationen, diverse Amyioidformationen und Läsionen des zerebralen Gefäß-Bindegewebs-Apparates.

Die oben genannten Alterationen betreffen insbesondere Personen, die im weiteren Sinne von der Alzheimer-Krankheit betroffen sind. Dies bedeutet, daß zum einen bei einer vorhandenen Alzheimer-Krankheit die oben genannten Alterationen besonders intensiv auftreten, zum anderen können die oben genannten Alterationen die Symptomatik der Alzheimer-Krankheit verstärken oder deren Auftreten begünstigen. Läsionen des cerebralen Gefäß-Bindegewebs-Apparates sind z.B. die Ursachen der Multi-Infarkt-Demenz (MID). Über die Ursachen der Multiplen Sklerose (MS) wird gegenwärtig noch heftig diskutiert.

Als weiteres neurologisches Leiden ist Morbus-Parkinson zu nennen. Diese Erkrankung tritt zusammen mit einem ausgeprägten Dopaminmangel im Bereich der Substantia nigra und im Striatum auf. Ursache dieser Erkrankung ist eine Degeneration von dopaminergen nigrostriären Projektionsneuronen. Auch bei dieser Krankheit wurden Demenzerscheinungen beschrieben.

Neben den bereits erwähnten Demenz-Erkrankungen treten aber auch eine Vielzahl weiterer allgemeiner Abnutzungserkrankungen auf; z.B. "impaired brain function of old age" (IBFO). Die Wahrscheinlichkeit des Auftretens von Schlaganfällen wird ebenfalls mit zunehmendem Alter größer. Weiterhin sind Krankheiten auf der Basis arteriosklerotischer Gefäßveränderungen zu nennen.

Ein weiterer Effekt, der mit zunehmendem Alterungsprozeß stärker auftritt, ist eine verminderte Adaptionsfähigkeit des "Streß-Managing-Systems". Dieses körpereigene System zur Streßbewältigung ist im Bereich Hypothalamus-Hypohyse-Nebennieren angesiedelt. Die Wirkungsweise dieses Streßbewältigungssystems beruht im wesentlichen darauf, daß der menschliche Körper bei einem Auftreten von Verletzungen, Infektionen oder psychosozialen Streß zum Beispiel mit der Freisetzung einer Vielzahl von Mediatoren reagiert, die aufeinander fein abgestimmt die Homöostase wiederherstellen.

Die Mediatoren sorgen u.a. dafür, daß eine gesteigerte Zellaktivität wieder reduziert wird, um eine Dauerüberlastung der Zellen zu vermeiden, die zu Zellschäden und im Extremfall zum Absterben der Zellen führen könnte.

Ein Beispiel für derartige Mediatoren sind die Steroid-Hormone. Diese Steroid-Hormone binden spezifisch an Rezeptoren, die im Bereich des Zellkernes angeordnet sind. Durch diese Anbindung an die Rezeptoren werden ein breites Spektrum von metabolischen Prozessen induziert. Darüber hinaus treten eine Reihe von neuroendokrino-immunologischen Interaktivitäten auf, die die Integrität und die Gesundheit des betroffenen Individuums sicherstellen.

Aus dem Artikel von **G. Delitala et al. in IRCS Med. Sci.-Clin. Med., 4,3, 1976, S. 113**, ist es bekannt, Clomiphene oral zu verabreichen, um den Funktionszustand der Hypothalamus-Hypophyse-Gonaden Achse bei Parkinson-Patienten zu testen. Als Ergebnis wurde eine partielle Toleranzentwicklung der Hypothalamus-Hypophysen Achse bei unbehandelten Parkinson-Personen festgestellt.

Im Artikel von **J.J. Oram et al. im Int. J. Geriatr. Psychiatry, 2,2, 1987, S. 125-126** wird ausgeführt, daß Patienten mit Alzheimer Demenz ein Neurotransmitterdefizit aufweisen: TRH und LHRH sind in der Zerebrospinalflüssigkeit erniedrigt. In einem Substitutionsversuch wird untersucht, ob TRH und LHRH die mentale Funktion verbessern können. Da das LHRH-Hormon offenbar nicht verfügbar war, wurden die Blutspiegel von LHRH **indirekt** angehoben durch die orale Gabe von Clomiphene Tabletten (Zit.: Clomiphene to raise cerebral levels of LHRH). In Abb. 1 sind die Ergebnisse dargestellt. Daraus folgt, daß nur das sog. "double active drug regime" aus TRH und CLOMIPHENE bzw. LHRH einen positiven Trend bezüglich der Verbesserung kognitiver Funktionen zeigt. CLOMIPHENE allein mit Plazebo hat hier **keinen** nachweisbaren Effekt auf die kognitiven Funktionen der Demenz-Patienten.

Im Artikel von J. Libiger und T. A. Ban in - **Prog. Neuro-Psychopharmacol.; 4, 1980, S. 561-567** wird ebenfalls die Wirkung von Clomifene untersucht. Eine überwachte Person zeigte nach Absetzen der Clomiphene Therapie eine sofortige Remission ihrer psychopathologischen Symptome, war also nur vorübergehend und reversibel erkrankt.

Bislang ist kein Präparat bekannt, das mit geringen Nebenwirkungen einem Auftreten von altersbedingten Abnutzungserscheinungen vorbeugen kann bzw. bei einer bereits aufgetretenen Erkrankung einem Fortschreiten der Erkrankung entgegenwirkt bzw. die Erkrankung therapiert.

Aufgabe der vorliegenden Erfindung ist es daher, ein Präparat der einleitend genannten Art derart anzugeben, daß bei der Therapie und Prophylaxe von Demenz-Erkrankungen eine hohe Wirksamkeit bei geringen Nebenwirkungen erreicht wird.

Diese Aufgabe wird erfindungsgemäß dadurch gelöst, daß als Wirkstoff Tamoxifen, 4-OH-Tamoxifen, 3-OH-Tamoxifen, N-Desmethyltamoxifen, Monophenoltamoxifen oder Cyanotamoxifen enthalten ist.

Durch das Einbringen von Tamoxifen oder den aufgeführten Tamoxifen-Derivaten als Zellkernrezeptoren-Blocker oder als Modulatoren in der Steroid-Rezeptor-Superfamilie werden Mechanismen aktiviert, die interaktive Beziehungen zwischen offensichtlich nicht verwandten Systemen induzieren. Durch diese Induktion ist es möglich, stimulierende Effekte auf ein gealtertes oder alteriertes Neuroendokrinium zu erzielen. Hieraus resultiert die Möglichkeit einer Modulation und Therapie von Nervensystemläsionen.

Die nachfolgend aufgeführten Verbindungen können als pure oder partielle Steroid-Antagonisten klassifiziert werden. Ein derartiger partieller Steroid-Antagonist ist insbesondere Tamoxifen. Tamoxifen hat zusätzlich eine inhibitorische Wirkung auf L- und N-Typ Ca²⁺-Kanäle. Durch einen derartigen Kalzium-Antagonismus können zu hohe intrazelluläre Ca²⁺-Spiegel verhindert werden, die einen Zelltod zur Folge haben könnten. Dies bedeutet, daß Tamoxifen neuroprotektiv wirkt.

Darüber hinaus ist auch ein Einfluß von Tamoxifen auf CA²⁺-aktivierte K⁺-Kanäle festzustellen. Bezüglich dieser Kanäle liegen Indizien vor, daß mit einem Einfluß auf Lern- und Gedächtnisleistungen zu rechnen ist. Ein Ligand wie Tamoxifen oder die genannten Tamoxifen-Derivate, die auf beide Kanaltypen wirken, können somit akut Lern- und Gedächtnisleistungen positiv beeinflussen und chronisch antidegenerativ wirken. Es handelt sich somit bei einer derartigen Substanz um ein ideales Mittel gegen die Alzheimer-Demenz. Dieses Wirkungsprinzip von Tamoxifen ist auch bei den anderen genannten Steroid-Antagonisten festzustellen. Von Antiöstrogenen ist bekannt, daß sie antioxidative Eigenschaften besitzen. Sie vermindern die Membranfluidität und stabilisieren somit Zellmembranen gegen Lipidperoxidation.

Die grundsätzliche Wirkung der beschriebenen Antiöstrogene resultiert also sowohl daraus, daß sie Liganden für Ca²⁺ - und K⁺-Kanäle sind, als auch daraus, daß sie antioxidative Eigenschaften besitzen. Hieraus kann eine prophylaktische und/oder eine therapeutische Wirkung abgeleitet werden.

Bezüglich der bereits erwähnten Steroid-Antagonisten ist zwischen puren Antagonisten und partiellen Agonisten zu unterscheiden. Ein purer Agonist weist keine intrinsischen antagonistischen Eigenschaften auf und ist für einen speziellen Rezeptor geeignet. Antagonisten, die partiell als Agonisten wirken, sind mindestens in einem Bereich ihres Anwendungsspektrums rezeptorunspezifisch und können je nach Art des Rezeptors unterschiedliche Wirkungen entfalten. Die Wirkung ist somit davon abhängig, im Bereich welchen Organes oder welchen Körperteiles die Wirkung entfaltet wird. Zusätzlich sind auch die Metaboliten der oben erwähnten Substanzen einsetzbar.

Die hier betrachtete Untergruppe der Steroid-Antagonisten bilden die Anti-Östrogene, die ebenfalls mit purem oder partiellem Eigenschaftsspektrum versehen sein können und die im weiteren genannten diesen zugeordneten Metaboliten und Abkömmlinge.

Tamoxifen und eine Vielzahl geeigneter Tamoxifen-Derivate werden in der Veröffentlichung "Comparative Affinity of Steroidal and Nonsteroidal Antioestrogens, Cholesterol Derivatives and Compounds with a Dialkylamino Side Chain for the Rat Liver Antioestrogen Binding Site" Biochemical Pharmacology, Vol. 43, No. 12, pp. 2511-2518,1992., C.D.M.A. van den Koedijk, C. Vis van Heemst, G.M. Elsendoorn, J.H.H. Thijssen and M.A. Blankenstein, beschrieben. Erfindungsgemäß werden Tamoxifen, 4-OH-Tamoxifen, 3-OH-Tamoxifen, N-Desmethyltamoxifen, Monophenoltamoxifen oder, Cyanotamoxifen eingesetzt.

Bei der akuten Wirkung des Präparates tritt zunächst eine Wechselwirkung mit den Rezeptoren auf, die eine Änderung der Zellaktivität zur Folge hat und darüber hinaus Zellsubstanzen freisetzt, die eine Trennung des Liganden vom Rezeptor begünstigen. Nach einer entsprechenden Trennung wird der Ligand im Rahmen des normalen Stoffwechsels abgebaut. Zur Aufrechterhaltung oder Wiederauffrischung der Wirkung ist es somit erforderlich, in bestimmten zeitlichen Abständen eine neue Dosis des Präparates zu applizieren.

## Patentansprüche

1. Verwendung eines Antiöstrogens, ausgewählt aus der Gruppe Tamoxifen, 4-OH-Tamoxifen, 3-OH-Tamoxifen, N-Desmethyltamoxifen, Monophenoltamoxifen und Cyanotamoxifen, zur Herstellung eines Arzneimittels zur Therapie und Prophylaxe von Demenzerkrankungen, insbesondere zur Therapie und Prophylaxe von regressiven Zellveränderungen.

## Claims

1. The use of an antioestrogen, selected from the group comprising tamoxifen, 4-OH-tamoxifen, 3-OH-tamoxifen, N-desmethyltamoxifen, monophenoltamoxifen and cyanotamoxifen, for the preparation of a medicament for the therapy and prophylaxis of dementia diseases, in particular for the therapy and prophylaxis of regressive cell changes.

## Revendications

1. Utilisation d'un anti-oestrogène choisi dans le groupe constitué par le tamoxifène, le 4-OH-tamoxifène, le 3-OH-tamoxifène, le N-desméthyltamoxifène, le monophénoltamoxifène et le cyanotamoxifène, pour la préparation d'un médicament destiné au traitement et à la prophylaxie des maladies associées à la démence, plus particulièrement pour le traitement et la prophylaxie des transformations cellulaires régressives.
